# EUROPEAN PATENT SPECIFICATION

(11) **EP 2 280 725 B1**
(45) Date of publication and mention of the grant of the patent: **23.05.2012**
(21) Application number: 09724781.1
(22) Date of filing: 25.03.2009
(51) Int. Cl.: A61K 39/102

(54) **VACCINE FOR PROTECTION AGAINST HAEMOPHILUS PARASUIS SEROTYPE 4 IN PIGLETS**
IMPFSTOFF ZUM SCHUTZ VOR HAEMOPHILUS PARASUIS SEROTYP 4 BEI FERKELN
VACCIN POUR PROTECTION CONTRE HAEMOPHILUS PARASUIS SÉROTYPE 4 CHEZ DES PORCELETS

(30) Priority: 26.03.2008 EP 08153290; 26.03.2008 US 39590 P
(43) Date of publication of application: 09.02.2011
(73) Proprietor: Intervet International B.V., 5831 AN Boxmeer (NL)
(72) Inventor: SEGERS, Ruud, Philip, Antoon, Maria, NL-5831 AN Boxmeer (NL); WITVLIET, Maarten, Hendrik, NL-5831 AN Boxmeer (NL); HENSEN, Selma, Marianne, NL-5831 AN Boxmeer (NL)
(74) Representative: Janssen, Paulus J. P.
(86) International application number: PCT/EP2009/053490
(87) International publication number: WO 2009/118330

(56) References cited:
- WO-A-00/01408
- SOLANO-AGUILAR G I ET AL: "Protective role of maternal antibodies against Haemophilus parasuis infection" AMERICAN JOURNAL OF VETERINARY RESEARCH, vol. 60, no. 1, January 1999 (1999-01), pages 81-87, XP009105166 ISSN: 0002-9645
- RAPP-GABRIELSON ET AL.: "HAEMOPHILUS PARASUIS : IMMUNITY IN SWINE AFTER VACCINATION" VETERINARY MEDICINE, January 1997 (1997-01), pages 83-90, XP009105182
- OLIVEIRA S. ET AL.: "EVALUATION OF HAEMOPHILUS PARASUIS CONTROL IN THE NURSERY USING VACCINATION AND CONTROLLED EXPOSURE" JOURNAL OF SWINE HEALTH AND PRODUCTION, vol. 12, no. 3, May 2004 (2004-05), pages 123-128, XP009105190
- BAK H ET AL: "Protection of vaccinated pigs against experimental infections with homologous and heterologous Haemophilus parasuis" VETERINARY RECORD, BRITISH VETERINARY ASSOCIATION, LONDON, GB, vol. 151, no. 17, 26 October 2002 (2002-10-26), pages 502-505, XP009101823 ISSN: 0042-4900
- KARG G ET AL: "The effect of season and vaccination for Glasser's disease and post-weaning colibacillosis in an outdoor pig unit endemically infected with virulent strain of Haemophilus parasuis serotype 5 and pathogenic Escherichia coli." JOURNAL OF VETERINARY MEDICINE SERIES B, vol. 49, no. 10, December 2002 (2002-12), pages 464-468, XP002494228 ISSN: 0931-1793

## Description

The present invention pertains to a vaccine to protect piglets against *Haemophilus parasuis* serotype 4.

*Haemophilus parasuis* is an opportunistic pathogenic bacterium of the upper respiratory tract of pigs. It is the cause of Glässer's disease, a condition that results in a wide variety of clinical signs such as arthritis, pericarditis, pleuritis, polyserositis, meningitis and acute death. Glässer's disease is mainly a problem associated with the nursery of piglets. However, with the provision of new health technologies such as segregated early weaning (SEW) and the emergence of new pathogens such as porcine reproductive and respiratory syndrome (PRRS) virus, the incidence of Glässer's disease has increased. Firstly, *Haemophilus parasuis* problems are exacerbated by PRRS infection. Next to this, although SEW technologies have eliminated *Haemophilus parasuis* from certain herds, this has become a very serious problem for breeding stock companies: they aim to elevate health status of their animals and in the process increase the susceptibility of replacement stock to *Haemophilus parasuis.* Acute Glässer's disease is the frequent result when high-health-status replacement gilts or boars are introduced onto an infected farm. Therefore, among other things it has become of major importance to immunize naïve animals before introducing them onto infected farms. Generally speaking, obtaining protection against *Haemophilus parasuis* via immunization has become increasingly important. There are different serotypes known of *Haemophilus parasuis*, each of these can be identified using the technique of immunodiffusion (Kielstein et al. in J. Clin. Microbiol. 30:862-865; 1992 and Rapp-Gabrielson et al. in AJVR 53:659-664; 1992)

Combinord® is a registered vaccine (Intervet Denmark) to protect (i.e. to at least mitigate a negative effect of a disorder) sows and gilts against *Haemophilus parasuis* serotype 5. It also passively protects the offspring against serotype 5 via intake of the colostrum. However, it is not registered for protection against *Haemophilus parasuis* serotyer 4. Indeed, as is generally known for vaccines that actively protect against *Haemophilus parasuis,* there is no cross protection of serotype 5 against serotype 4. This is known i.a. from a publication by Rapp-Gabrielson et al (Veterinary Medicine/January 1997/pp 83-90). Rapp-Gabrielson has examined differences in virulence and immunogenicity of strains of *Haemophilus parasuis,* focussing on protection and cross-protection against strains representing prevalent serotypes, viz. serotypes 5, 4, 13, 14, 2 and 12 respectively. Several conclusions could be drawn from this research. Most importantly, it was shown that *Haemophilus parasuis* serotype 5 provided no protection against serotype 4 challenge. Next to this, it was shown that within serotype 4, cross protection against heterologous strains was provided. Thus, protection against a strain of serotype 4 implied protection against other strains of the same serotype 4.

The above findings are in line with the findings by Oliveira (Journal of Swine Health and Production, Volume 12, Number 3, May and June 2004, pp 123-128. Here, it was established that with *Haemophilus parasuis*, there is a lack of cross-protection between different serotypes. Recently (March 21, 2006), Fort Dodge was granted marketing authorisation in the United Kingdom for a vaccine against *Haemophilus parasuis* serotypes 4 and 5 (Suvaxyn M.hyo - Parasuis). Indeed, the vaccine comprises both inactivated *Haemophilus parasuis* serotype 4 as well as *Haemophilus parasuis* serotype 5. The UK based RUMA (Responsible Use of Medicines in agriculture Alliance) has published guidelines on the use of vaccines and vaccination in pig production in November 2006. With respect to Glässer's disease these guidelines state that the commercial vaccines rely on strains from which there is little or no cross immunity to others.

The disadvantage of hitherto known vaccines is that for protection against the serotypes that are most prevalent, i.e. serotypes 4 and 5, both serotypes have to be in the vaccine. This is disadvantageous from a safety point of view (more bacteria present means a higher LPS content of the vaccine), but also from an economical point of view: the presence of two types of bacteria in the vaccine implies a higher chance of batch failure and a higher risk of contamination. Therefore, the chance of a batch being found unfit for sales is increased. It is therefore an object of the present invention to overcome or at least mitigate the disadvantages of the present vaccines against *Haemophilus parasuis.*

To this end it has been found that one can make use of *Haemophilus parasuis* serotype 5 bacteria in the manufacture of a vaccine for administration to a pregnant sow or gilt, to protect a piglet through the intake of colostrum of the said sow or gilt after she has farrowed against a disorder arising from *Haemophilus parasuis* serotype 4 bacteria. Applicant has shown that when the sow or gilt is vaccinated with a vaccine based on antigens of *Haemophilus parasuis* serotype 5 bacteria, a piglet is protected against a disorder arising from *Haemophilus parasuis* serotype 4 bacteria when the piglet takes colostrum of the said sow or gilt in after she has farrowed. Given the prior art that specifically and persistently teaches that a vaccine containing antigens of *Haemophilus parasuis* serotype 5 bacteria does not provide protection against *Haemophilus parasuis* serotype 4 bacteria this could not have been reasonably expected. Note that many embodiments for the use according to the present invention can be devised, once knowing that cross protection against serotype 4 can be provided by immunising the sow or gilt against *Haemophilus parasuis* serotype 5. For example, the bacteria could be put in the vaccine as such (either live attenuated or killed), the bacteria could be used to obtain an extract of one or more immunogenic components of the bacterium to put in the vaccine, or they could be used to arrive even at a recombinant subunit of the bacterium to be used as the actual antigen to formulate the vaccine itself.

In an embodiment the vaccine comprises inactivated cells of *Haemophilus parasuis* serotype 5 bacteria and/or derivatives thereof. It appears that a vaccine comprising inactivated cells and/or derivatives thereof provides sufficient protection. The advantage of inactive component is safety. The *Haemophilus parasuis* serotype 5 bacteria may be inactivated by any art-known method, such as by using chemical inactivators such as beta-propiolactone, thimerosal or (another mercury donating agent), formaldehyde etc., applying physical methods such as heat, UV-light, micro-waves etc., by using biological methods such as enyzme-based methods to kill the bacteria, and any other method as is commonly applied in the art. By using such methods, parts of the bacterial cells may lose their association with the cells. In particular, this might be the case with cell membrane associated components such as the outer membrane itself or outer membrane proteins. This way, the non-whole killed cells that remain can be regarded as derivatives in the sense of the present invention. Also, the parts that lost their association with the cells can be regarded as derivatives in the sense of the present invention. It is commonly known, in particular for outer membrane associated components, that these components can contribute in large to the effective immunological response of the target animal to the vaccine. As such, these components could be used in many cases as the sole components in the vaccine. In the latter case, one can use various art-known methods to obtain such component in (substantially) pure form, e.g. by having it made via a recombinant technique, by synthesizing the component or by purification of the component out of fermentation broth. In general, a derivative in the sense of the present invention is a non-live component of *Haemophilus parasuis* bacteria, other than inactivated whole cells.

In another embodiment a first vaccination of the sow or gilt is followed by a second vaccination, the first and second vaccination taking place before the sow or gilt has farrowed. This vaccination scheme has provided to lead to optimal protection of the piglet. In a preferred embodiment the first vaccination takes place 6 to 8 weeks before an expected date of farrowing and the second vaccination takes place 2 to 4 weeks before this date.

The invention also pertains to *Haemophilus parasuis* serotype 5 bacteria for use in the manufacture of a vaccine for administration to a pregnant sow or gilt, to protect piglets through the intake of colostrum of the said sow or gilt after she has farrowed against a disorder arising from *Haemophilus parasuis* serotype 4 bacteria.

The invention will be explained by the following examples that pertain to a preferred embodiment of the present invention.

### EXPERIMENTAL DESIGN

Five sows (SPF pigs, *H. parasuis* free) were vaccinated (intramuscular, 2 ml) with the commercially available vaccine Porcilis Glässer DF at 6 to 8 weeks before the expected date of farrowing and 4 weeks later (again i.m., 2 ml). This vaccine is available from Intervet B.V., a part of Schering-Plough Corporation, Boxmeer, the Netherlands. The vaccine comprises formaldehyde inactivated whole cells of *Haemophilus parasuis* serotype 5 bacteria and the active component is suspended in Diluvac Forte (oil-in-water) adjuvant. In general, any other vaccine comprising *H. parasuis* serotype 5 antigens can be formulated by using art-known methods that basically comprise admixing suitable antigens of *H. parasuis* (live or inactivated, whole cell, extract, purified fraction or even subunit) with a pharmaceutically acceptable carrier, e.g. a liquid carrier such as (optionally buffered) water or a solid carrier such as commonly used to obtain freeze-dried vaccines. Optionally other substances such as adjuvants, stabilisers, viscosity modifiers or other components are added depending on the intended use or required properties of the vaccine. In the present example we used the commercially available vaccine as mentioned here-above.

Five non-vaccinated sows were included as negative controls. At approximately 4 weeks of age, 2 piglets from each sow were transferred to the experimental farm and were challenged with *H. parasuis.* Post mortem examination was performed at 10 days after challenge or earlier in case the animal died or had to be euthanized for animal welfare reasons. Blood and colostrum samples were collected of the sows around farrowing.

The challenge compound is a field isolate (live strain) of *Haemophilus parasuis* serotype 4. The isolate is obtained by using a method as known from the Oliveira reference as mentioned here-above. The challenge culture was freshly prepared prior to challenge. *Haemophilus parasuis* was cultured on chocolate agar plates with NAD at 37° for 24-72 hours. Bacteria were harvested with 2 to 5 ml CYS medium per plate and this was used to inoculate CYS medium with NAD (2 to 5 ml per 100 ml medium). This was followed by incubation at 37°C for ± 5 hours. Then, the culture was centrifuged for 10 min at 2.000 g and the pellet was resuspended in 0.04 M PBS. The challenge materials was stored on ice until use, which use was within 2 hours. The piglets were challenged with *Haemophilus parasuis* by the aerosol route in an aerosol box. This was done in two groups of 10 piglets each (5 passively immunized piglets and 5 controls). The aerosol was given by means of a Devilbis Nebulizer. The total amount of challenge culture per group of 10 piglets was 50 ml. The challenge culture contained ± 10⁸ cfu/ml. The piglets were left in the aerosol box for approximately 30 minutes.

After challenge, rectal temperature, general condition, locomotion, nervous system and other abnormalities were scored daily.

The scoring system for the general condition was as follows
0= normal
1= inappetant
2= inappetant and depressed
3= depressed and slow to rise
4= moribund.

The scoring system for the locomotion was as follows:
0= normal behavior
1= swollen, hot or painful joints
2= limping on one or more legs
3= failure to put a foot to the ground
4= refusal to stand if not moribund.

The scoring system for the nervous system was as follows:
0= normal behavior
1= tilted head
2= loss of balance
4= convulsions

The scoring system for other abnormalities was as follows:
0= no abnormalities
2= minor abnormalities
4= severe abnormality requiring euthanasia.

Any piglet that was euthanized was automatically given a score of 4 for the category of clinical sign that led to the humane endpoint decision. The total clinical score per piglet was the sum of daily clinical scores from challenge to post mortem examination.

### RESULTS

Sows showed a good increase in serum titres against *H. parasuis* after vaccinations with Porcilis Glässer. Before vaccination serum titres of all sows were below 6.7 log₂, except for one sow of the vaccinated group which had a serum titre of 8.4 log₂ before first vaccination. Serum titres just before farrowing ranged from 8.6 to 12.6 log₂ for the vaccinated sows, while titres of the non-vaccinated sows remained below the detection limit of the ELISA (<6.7 log₂). Titres in colostrum of vaccinated sows ranged from 9.0 to >13.7 log₂, while titres in the colostrum of non-vaccinated sows ranged from <6.6 to 7.9 log₂. At 4 weeks of age, 2 piglets from each sow were challenged with *H. parasuis* serotype 4. The results are presented in table 1.

Table 1 shows that the mean clinical score (i.e. the mean value for all 10 pigs of the total clinical score per piglet, i.e. the sum of daily clinical scores from challenge to post mortem examination) for the 10 piglets that have taken in colostrum from vaccinated pigs is far less than the mean clinical score for the control animals. The same is true for mortality.

**Table 1 Results for protection of the piglets**

| Treatment | No of piglets | Mean clinical score | Mortality [n/nₜₒₜ] |
|---|---|---|---|
| Passively immunized | 10 | 1,2 | 0/10 |
| Controls | 10 | 48 | 6/10 |

In the study here described significant reductions of clinical signs and mortality in the passively immunized piglets as compared to the control piglets after challenge were observed. The sow that already had a serum titre against *H. parasuis* at the start of the experiment responded well to vaccination as the serum titre increased. The results after challenge of the two piglets of this sow were completely comparable to the other piglets of the same group. This is an indication that the higher titre of the sow at the start of the study had no influence on the outcome of the study. To verify that there was no effect on the outcome of the study, the statistics were repeated after excluding the results of the 2 piglets derived from this sow. Clinical signs and mortality were again significantly different from those of the control group. Altogether, it can be concluded that by using *Haemophilus parasuis* serotype 5 bacteria to manufacture a vaccine for administration to a pregnant sow or gilt, one can protect a piglet through the intake of colostrum of the said sow or gilt after she has farrowed against a disorder arising from *Haemophilus parasuis* serotype 4 bacteria.

## Claims

1. Use of *Haemophilus parasuis* serotype 5 bacteria in the manufacture of a vaccine for administration to a pregnant sow or gilt, to protect a piglet through the intake of colostrum of the said sow or gilt after she has farrowed, against a disorder arising from *Haemophilus parasuis* serotype 4 bacteria.

2. Use according to claim 1, **characterised in that** the vaccine comprises inactivated cells of *Haemophilus parasuis* serotype 5 bacteria and/or derivatives thereof.

3. Use according to any of the preceding claims, **characterised in that** a first vaccination of the sow or gilt is followed by a second vaccination, the first and second vaccination taking place before the sow or gilt has farrowed.

4. Use according to claim 3, **characterised in that** the first vaccination takes place 6 to 8 weeks before an expected date of farrowing and the second vaccination takes place 2 to 4 weeks before this date.

5. *Haemophilus parasuis* serotype 5 bacteria for use in the manufacture of a vaccine for administration to a pregnant sow or gilt, to protect piglets through the intake of colostrum of the said sow or gilt after she has farrowed, against a disorder arising from *Haemophilus parasuis* serotype 4 bacteria.

## Patentansprüche

1. Verwendung von *Haemophilus parasuis* Serotyp-5-Bakterien bei der Herstellung eines Impfstoffs zur Verabreichung an eine trächtige Sau oder Jungsau zum Schutz eines Ferkels durch die Aufnahme von Kolostralmilch dieser Sau oder Jungsau nach dem Abferkeln gegen eine Erkrankung, die durch *Haemophilus parasuis* Serotyp-4-Bakterien verursacht wird.

2. Verwendung nach Anspruch 1, **dadurch gekennzeichnet, dass** der Impfstoff inaktivierte Zellen von *Haemophilus parasuis* Serotyp-5-Bakterien und/oder Derivate davon umfasst.

3. Verwendung nach einem der vorhergehenden Ansprüche, **dadurch gekennzeichnet, dass** auf eine erste Impfung der Sau oder Jungsau eine zweite Impfung folgt, wobei die erste und die zweite Impfung stattfinden, bevor die Sau oder Jungsau abgeferkelt hat.

4. Verwendung nach Anspruch 3, **dadurch gekennzeichnet, dass** die erste Impfung 6 bis 8 Wochen vor einem erwarteten Abferkeltermin und die zweite Impfung 2 bis 4 Wochen vor diesem Termin stattfindet.

5. *Haemophilus parasuis* Serotyp-5-Bakterien für die Verwendung bei der Herstellung eines Impfstoffs zur Verabreichung an eine trächtige Sau oder Jungsau zum Schutz eines Ferkels durch die Aufnahme von Kolostralmilch dieser Sau oder Jungsau nach dem Abferkeln gegen eine Erkrankung, die durch *Haemophilus parasuis* Serotyp-4-Bakterien verursacht wird.

## Revendications

1. Utilisation de bactéries *Haemophilus parasuis* sérotype 5 dans la fabrication d'un vaccin destiné à l'administration à une truie ou jeune truie pleine, pour protéger un porcelet grâce à la consommation du colostrum de ladite truie ou jeune truie après la mise bas, contre un trouble provenant de bactéries *Haemophilus parasuis* sérotype 4.

2. Utilisation selon la revendication 1, **caractérisée en ce que** le vaccin comprend des cellules inactivées de bactéries *Haemophilus parasuis* sérotype 5 et/ou de dérivés de celles-ci.

3. Utilisation selon l'une quelconque des revendications précédentes, **caractérisée en ce qu'**une première vaccination de la truie ou jeune truie est suivie d'une deuxième vaccination, les première et deuxième vaccinations ayant lieu avant la mise bas de la truie ou jeune truie.

4. Utilisation selon la revendication 4, **caractérisée en ce que** la première vaccination a lieu 6 à 8 semaines avant une date prévue de mise bas et la deuxième vaccination a lieu 2 à 4 semaines avant cette date.

5. Bactérie *Haemophilus parasuis* sérotype 5, destinée à être utilisée dans la fabrication d'un vaccin destiné à l'administration à une truie ou jeune truie pleine, pour protéger un porcelet grâce à la consommation du colostrum de ladite truie ou jeune truie après la mise bas, contre un trouble provenant de bactéries *Haemophilus parasuis* sérotype 4.
